# EUROPEAN PATENT APPLICATION

(11) **EP 3 919 612 A1**
(43) Date of publication of application: **08.12.2021**
(21) Application number: 19912987.5
(22) Date of filing: 19.12.2019
(51) Int. Cl.: C12N 5/074

(54) **COMPOSITION AND APPLICATION THEREOF**

(30) Priority: 30.01.2019 CN 201910091150
(71) Applicant: Guangzhou Institutes of Biomedicine and Health, Chinese Academy of Sciences, Guangzhou, Guangdong 510530 (CN)
(72) Inventor: LI, Yinxiong, Guangzhou, Guangdong 510530 (CN); ZHUANG, Yuanqi, Guangzhou, Guangdong 510530 (CN); PAN, Tingcai, Guangzhou, Guangdong 510530 (CN); CHEN, Yan, Guangzhou, Guangdong 510530 (CN)
(74) Representative: Germain Maureau
(86) International application number: PCT/CN2019/126579
(87) International publication number: WO 2020/155910

(57) **Abstract**

Provided is a composition and an application thereof. The composition is used to prepare a medium for inducing differentiation of human pluripotent stem cells to liver precursor cells. By means of screening active components, optimizing the composition ratio and adding a GSK3-beta inhibitor, a Nodal activator, a BMP activator, a BMP inhibitor and a Hedgehog activator, human pluripotent stem cells are induced to differentiate to liver precursor cells. The process is simple and efficient, the content of positive cells is high, and the cost of cell differentiation is reduced. The invention can be used for research and application in drug development and regenerative medicinal treatment, and has broad application prospects.

## Description

### TECHNICAL FIELD

The present application belongs to the field of biotechnology, relates to a composition and use thereof and, in particular, to a composition and use thereof in the preparation of a medium for inducing human pluripotent stem cells differentiation into hepatic progenitor cells.

### BACKGROUND

Liver is the largest organ and gland of human body, which bears important endocrine and exocrine functions. At present, liver diseases have high morbidity and mortality. According to the report in 2014, there are more than 1.3 billion patients with liver diseases around the world, and there are as many as 300 million patients with liver diseases in China. Liver diseases include viral hepatitis, alcoholic steatohepatitis, and nonalcoholic steatohepatitis. End-stage liver diseases (liver cirrhosis and liver cancer) are irreversible. Moreover, clinically, no effective drugs have been found to treat liver cirrhosis and liver cancer. The above reasons lead to a high mortality rate of liver diseases.

At present, the effective method to treat end-stage liver diseases is liver transplantation. However, liver transplantation is limited by donor shortage and immune rejection. Researchers mainly adopt two methods to reduce the shortage of liver sources. In one aspect, researchers use hepatic cell transplantation to replace liver transplantation, which makes the operation simpler and the timing of use more flexible. However, it is difficult for primary hepatocytes to expand *in vitro* and maintain metabolic function. Therefore, researchers try to use stem cell-derived hepatic progenitor cells or hepatocyte-like cells instead of primary hepatocytes to treat liver injury. Hepatic progenitor cells have the ability of proliferation and the potential of hepatobiliary bidirectional differentiation. Therefore, transplantation of hepatic progenitor cells to treat liver diseases has gradually become a research hotspot in the field of regenerative medicine.

Hepatic progenitor cells are mainly obtained in the following methods: extracting from liver; and differentiating stem cells into hepatic progenitor cells. Due to the shortage of liver sources, it is difficult to obtain enough primary hepatic progenitor cells for clinical treatment. Since embryonic stem cells (ESC) and induced pluripotent stem cells (iPSC) have the potential of infinite proliferation and multidirectional differentiation, differentiating these cells into hepatic progenitor cells can solve the problem of shortage of liver sources.

The differentiation of hepatic progenitor cells can be divided into two stages: definitive endoderm induction (DE induction) and hepatic differentiation. Signaling pathways such as Wnt, Activin/Nodal, and PI3K play an essential role in the regulation in the process of DE induction. The stage of hepatic differentiation mainly depends on BMP and FGF signaling pathways.

Although the technology of using growth factors to differentiate stem cells into hepatic progenitor cells has been gradually improved, the extensive use of growth factors makes differentiation costly. In addition, growth factors are prone to inactivation during long-term storage, which affects the repeatability of experiments. The above reasons hinder the extensive clinical use of stem cell-derived hepatic progenitor cells.

Therefore, it is urgent to establish a new and efficient differentiation solution based on small molecule compounds in the art to reduce the cost of differentiation of stem cells into hepatic progenitor cells.

### SUMMARY

The summary of the subject matter described herein is given below in detail. This summary is not intended to limit the scope of the claims.

The present application provides a composition and use thereof. Human pluripotent stem cells are induced to differentiate into hepatic progenitor cells by adding the composition into differentiating cells. The process is simple, efficient and quick, and the cost of cell differentiation is reduced. The composition and use thereof can be used for research and application in drug development and regenerative medicinal treatment and have broad application prospects.

To achieve this object, the present application adopts technical solutions described below.

In a first aspect, the present application provides a composition comprising a basal medium, and further comprising any one or a combination of at least two of a GSK3-beta inhibitor, a Nodal agonist, a BMP agonist, a BMP inhibitor, a Hedgehog agonist or a serum substitute.

In the present application, in order to solve the disadvantage of inducing the differentiation of human pluripotent stem cells into hepatic progenitor cell in the related art, the inventor, after the long-term scientific research practice, induces human pluripotent stem cells to differentiate into hepatic progenitor cells by screening active components, optimizing the composition ratio, and adding a GSK3-beta inhibitor, a Nodal agonist, a BMP agonist, a BMP inhibitor, and a Hedgehog agonist into differentiating cells. The process is simple and efficient, the content of positive cells is high, and the cost of cell differentiation is reduced. The composition and application thereof can be used for research and application in drug development and regenerative medicinal treatment and have broad application prospects.

Preferably, the basal medium includes any one or a combination of at least two of RPMI 1640, Dulbecco's Modified Eagle Medium, Ham's F12, DMEM/F12, Ham's F-12K Medium, HepatoZYME-SFM, William's E Medium, Waymouth's Medium or Hepatocyte Culture Medium.

Preferably, the GSK3-beta inhibitor includes any one or a combination of at least two of CHIR99021 HCL, CHIR99021 or BIO, preferably CHIR99021 HCL.

Preferably, the concentration of CHIR99021 HCL is 0.5 µM to 4.5 µM, and can be, for example, 0.5 µM, 1 µM, 2 µM, 3 µM, 4 µM or 4.5 µM.

The concentration of CHIR99021 HCL in the final medium is 0.5 µM to 4.5 µM, and can be, for example, 0.5 µM, 1 µM, 2 µM, 3 µM, 4 µM or 4.5 µM.

Preferably, the Nodal agonist includes IDE1 and/or IDE2.

Preferably, the concentration of IDE1 is 0.5 µM to 4 µM, and can be, for example, 0.5 µM, 1 µM, 2 µM, 3 µM or 4 µM.

The concentration of IDE1 in the final medium is 0.5 µM to 4 µM, and can be, for example, 0.5 µM, 1 µM, 2 µM, 3 µM or 4 µM.

Preferably, the BMP agonist includes any one or a combination of at least two of isoliquiritigenin, 4-hydroxychalcone or kartogenin.

Preferably, the concentration of the BMP agonist is 1 µM to 20 µM, and can be, for example, 1 µM, 2 µM, 4 µM, 6 µM, 8 µM, 10 µM, 12 µM, 14 µM, 16 µM, 18 µM or 20 µM.

The concentration of 4-hydroxychalcone in the final medium is 1 µM to 20 µM, and can be, for example, 1 µM, 2 µM, 4 µM, 6 µM, 8 µM, 10 µM, 12 µM, 14 µM, 16 µM, 18 µM or 20 µM.

Preferably, the BMP inhibitor includes any one or a combination of at least two of dorsomorphin 2HCl, dorsomorphin or LDN-193189.

Preferably, the concentration of the BMP inhibitor, dorsomorphin 2HCl, is 0 to 1 µM and can be, for example, 0 µM, 0.2 µM, 0.4 µM, 0.6 µM, 0.8 µM or 1 µM.

The concentration of dorsomorphin 2HCl in the final medium is 0 µM to 1 µM, and can be, for example, 0 µM, 0.2 µM, 0.4 µM, 0.6 µM, 0.8 µM or 1 µM.

Preferably, the Hedgehog agonist includes Smoothened Agonist HCl.

Preferably, the concentration of the Hedgehog agonist is 0.2 µM to 1 µM, and can be, for example, 0.2 µM, 0.4 µM, 0.6 µM, 0.8 µM or 1 µM.

The concentration of Smoothened Agonist HCl in the final medium is 0.2 µM to 1 µM, and can be, for example, 0.2 µM, 0.4 µM, 0.6 µM, 0.8 µM or 1 µM.

Preferably, the serum substitute includes insulin-free B27 and/or insulin-containing B27.

Preferably, the volume concentration of the serum substitute is 0% to 3%, and may be, for example, 0%, 0.5%, 1%, 1.5%, 2%, 2.5% or 3%.

The small molecule compounds used in the final medium are shown in Table 1.

**Table 1 Chinese and English names and CAS numbers of small molecule compounds**

| English | Chinese | CAS |
|---|---|---|
| CHIR99021 HCL | 6-[[2-[[4-(2,4-dichlorophenyl)-5-(5-methyl-1H-imidazol-2-yl)-2-pyrimi dinyl]amino]ethyl]amino]-3-pyridinecarbonitrile hydrochloride | 179798 9-42-4 |
| IDE1 | 1-[2-[(2-carboxyphenyl)methylene]hydrazide]heptanoic acid | 116092 7-48-9 |
| Isoliquiritigenin | isoliquiritigenin | 961-29-5 |
| 4'-Hydroxychalcone | 4-hydroxychalcone | 2657-25-2 |
| Kartogenin | 2-([1,1-biphenyl]-4-ylcarbamoyl)benzoic acid | 4727-31 -5 |
| Dorsomorphin 2HCl | 6-[4-[2-(1-piperidinyl)ethoxy]phenyl]-3-(4-pyridyl)pyrazolo[1,5-A]pyri midine dihydrochloride | 121916 8-18-9 |
| Smoothened Agonist HCl | 3-chloro-N-[trans-4-(methylamino)cyclohexyl]-N-[[3-(4-pyridyl)pheny1]methyl]-benzo[B]thiophene-2-carboxamide | 912545-86-9 |

In a second aspect, the present application provides use of the composition described in the first aspect in the preparation of a cell culture medium for inducing differentiation of human pluripotent stem cells into hepatic progenitor cells.

Preferably, the human pluripotent stem cells include a human embryonic stem cell line and a human induced pluripotent stem cell line.

Preferably, the human embryonic stem cell line includes WA01 (HI) and/or WA09 (H9).

Preferably, the human induced pluripotent stem cell line includes UiPSC-013 (UC-013) and/or UiPSC-015 (UC-015).

The hepatic progenitor cells are cells obtained from the differentiation of pluripotent stem cells and expressing alpha fetoprotein (AFP) and hepatocyte nuclear factor 4α (HNF4α), and have the potential for differentiating into hepatic parenchymal cells.

The hepatic progenitor cells provided in the present application are hepatic progenitor cells which are obtained from the differentiation of human stem cells and express epithelial cell adhesion molecule (EpCAM), alpha fetoprotein (AFP) and hepatocyte nuclear factor 4α (HNF4α), and can differentiate into hepatic cells and bile duct cells *in vitro* and can also repair damaged liver of immunodeficient mice *in vivo.*

In a third aspect, the present application provides a cell culture medium for inducing differentiation of human pluripotent stem cells into hepatic progenitor cells, wherein the cell culture medium is prepared from the composition described in the first aspect.

Preferably, the cell culture medium includes any one or a combination of at least two of an endoderm induction medium I, an endoderm induction medium II, an endoderm induction medium III or a hepatic progenitor cell induction medium.

Preferably, the endoderm induction medium I contains insulin-free B27 with a volume concentration of 0 to 3%, CHIR99021 HCL with a concentration of 1 µM to 4.5 µM, IDE1 with a concentration of 0.5 µM to 4 µM, isoliquiritigenin with a concentration of 1 µM to 20 µM, and a basal medium; wherein the content of insulin-free B27 is preferably 1% to 2%, particularly preferably 2%; the concentration of CHIR99021 HCL is preferably 2 µM to 4 µM, particularly preferably 3 µM; the concentration of IDE1 is preferably 0.5 µM to 2 µM, particularly preferably 1 µM; and the concentration of isoliquiritigenin is preferably from 1 µM to 5 µM, particularly preferably 2.5 µM.

Preferably, the endoderm induction medium II contains insulin-free B27 with a volume concentration of 0 to 3%, CHIR99021 HCL with a concentration of 0.5 µM to 3 µM, IDE1 with a concentration of 0.5 µM to 4 µM, isoliquiritigenin with a concentration of 1 µM to 20 µM, and a basal medium; wherein the content of insulin-free B27 is preferably 1% to 2%, particularly preferably 2%; the concentration of CHIR99021 HCL is preferably 0.5 µM to 2 µM, particularly preferably 1 µM; the concentration of IDE1 is preferably 0.5 µM to 2 µM, particularly preferably 1 µM; and the concentration of isoliquiritigenin is preferably from 1 µM to 5 µM, particularly preferably 2.5 µM.

Preferably, the endoderm induction medium III contains insulin-free B27 with a volume concentration of 0 to 3%, CHIR99021 HCL with a concentration of 0.5 µM to 3 µM, IDE1 with a concentration of 0.5 µM to 4 µM, dorsomorphin 2HCl with a concentration of 0 to 1 µM, and a basal medium; wherein the content of insulin-free B27 is preferably 1% to 2%, particularly preferably 2%; the concentration of CHIR99021 HCL is preferably 0.5 µM to 2 µM, particularly preferably 1 µM; the concentration of IDE1 is preferably 0.5 µM to 2 µM, particularly preferably 1 µM; and the concentration of dorsomorphin 2HCl is preferably from µM to 0.5 µM, particularly preferably 0.2 µM.

Preferably, the hepatic progenitor medium contains B27 with a volume concentration of 0 to 3%, isoliquiritigenin with a concentration of 1 µM to 20 µM, 4-hydroxychalcone with a concentration of 1 µM to 20 µM, Smoothened Agonist HCl with a concentration of 0 to 2 µM, and a basal medium; wherein the content of B27 is preferably 1% to 2%, particularly preferably 2%; the concentration of isoliquiritigenin is preferably 2 µM to 10 µM, particularly preferably 2.5 µM; the concentration of 4-hydroxychalcone is preferably 2 µM to 10 µM, particularly preferably 2.5 µM; and the concentration of Smoothened Agonist HCl is preferably from 0 to 1 µM, particularly preferably 0.5 µM.

The basal medium of the endoderm induction medium is preferably RPMI 1640; and the basal medium of the hepatic progenitor medium is preferably HepatoZYME-SFM.

In a fourth aspect, the present application provides a method for inducing differentiation of pluripotent stem cells into hepatic progenitor cells, wherein the method adopts the cell culture medium described in the third aspect.

As a preferred technical solution, the method for inducing differentiation of pluripotent stem cells into hepatic progenitor cells specifically includes following steps:
(1) culturing pluripotent stem cells by using an endoderm induction medium I;
(2) culturing the cells obtained in step (1) by using an endoderm induction medium II;
(3) culturing the cells obtained in step (2) by using an endoderm induction medium III;
(4) culturing the cells obtained in step (3) by using a hepatic progenitor cell induction medium to obtain hepatic progenitor cells; and
(5) detecting and analyzing the obtained hepatic progenitor cells.

Preferably, a method for the detection and analysis in step (5) is to check the expression of a hepatic progenitor cell marker by fluorescence quantitative polymerase chain reaction (PCR), immunofluorescence or flow cytometry.

Preferably, the hepatic progenitor cell marker includes any one or a combination of at least two of AFP, HNF4α or EPCAM

In the preceding method, human pluripotent stem cells are cultured by using the endoderm induction medium I for 24 hours, the cells obtained in step (1) are cultured for 48 hours by using the endoderm induction medium II, the cells obtained in step (2) are cultured for 24 hours by using the endoderm induction medium III, and the cells obtained in step (3) are cultured for 5 days by using the hepatic progenitor cell induction medium.

In the present application, on the basis of the composition described in the first aspect, aiming at the technical difficulties of inducing pluripotent stem cells to differentiate into hepatic progenitor cell and the nutritional requirements of cell growth and differentiation, the inventor prepares four culture mediums and adopts different culture mediums at different stages, respectively. Each culture medium and culture time cooperate with each other, increasing synergy. The process is simple and efficient, the content of positive cells is high, and the cost of cell differentiation is reduced. The composition and use thereof can be used for research and application in drug development and regenerative medicinal treatment and have broad application prospects.

Compared with the related art, the present application has beneficial effects described below.

The induction method based on the cell culture medium prepared from the composition can differentiate human pluripotent stem cells into human hepatic progenitor cells in 9 days, and the induction efficiency is over 80%. The induced hepatic progenitor cells can further differentiate into hepatic cells or bile duct cells. The induced hepatocytes can express marker genes and proteins of hepatic cells, express P450 enzyme metabolic drugs, and store glycogen. The induction solution can improve the differentiation efficiency of human stem cells into hepatic progenitor cell, reduce the cost of differentiation, and lay a foundation for clinical use of stem cell-derived hepatic progenitor cells in the future.

Other aspects can be understood after the drawings and the detailed description are read and understood.

### BRIEF DESCRIPTION OF DRAWINGS

FIG. 1 is a graph showing morphological changes of cells during the differentiation process according to the present application;
FIG. 2A is a graph showing the expression of related marker genes by induced hepatic progenitor cells, detected by quantitative RT-PCR, according to the present application;
FIG. 2B is a graph showing the expression of marker protein alpha fetoprotein (AFP) by induced hepatic progenitor cells, detected by flow cytometry, according to the present application;
FIG. 2C is a graph showing the expression of marker protein AFP and hepatocyte nuclear factor 4α (HNF4α) by hepatic progenitor cells, detected by immunofluorescence, according to the present application;
FIG. 3A is a graph showing the expression of marker protein albumin (ALB) and alpha-antitrypsin (AAT) by hepatic cells, detected by immunofluorescence, according to the present application;
FIG. 3B is a graph showing results illustrating that hepatic cells have the capabilities to store glycogen, take-up indocyanine green (ICG), and take-up low density lipoprotein (LDL) according to the present application;
FIG. 3C is a graph showing the expression of related marker genes by hepatic cells, detected by quantitative RT-PCR, according to the present application;
FIG. 3D is a graph showing results illustrating that hepatic cells can express albumin, detected by ELISA test, according to the present application;
FIG. 3E is a graph showing results illustrating that hepatic cells have CYP3A4 activity, detected by mass spectrometry, according to the present application;
FIG. 3F is a graph showing results illustrating that hepatic cells have CYP2C9 activity, detected by mass spectrometry, according to the present application;
FIG. 3G is a graph showing results illustrating that cells have CYP2D6 activity, detected by mass spectrometry, according to the present application;
FIG. 4A is a graph showing results of the expression of related marker genes by bile duct cells, detected by quantitative RT-PCR, according to the present application;
FIG. 4B is a diagram showing the expression of marker protein actin microfilament (F-ACTIN) and cytokeratin 19 (CK19) by bile duct cells, detected by immunofluorescence, according to the present application;
FIG. 5A is a graph showing transplantation results of EPCAM+ & C-KIT- cells sorted by flow cytometry according to the present application;
FIG. 5B is a graph showing results of hepatic cells which can be used for detecting the expression of human albumin in mouse liver 7 days after transplantation according to the present application; and

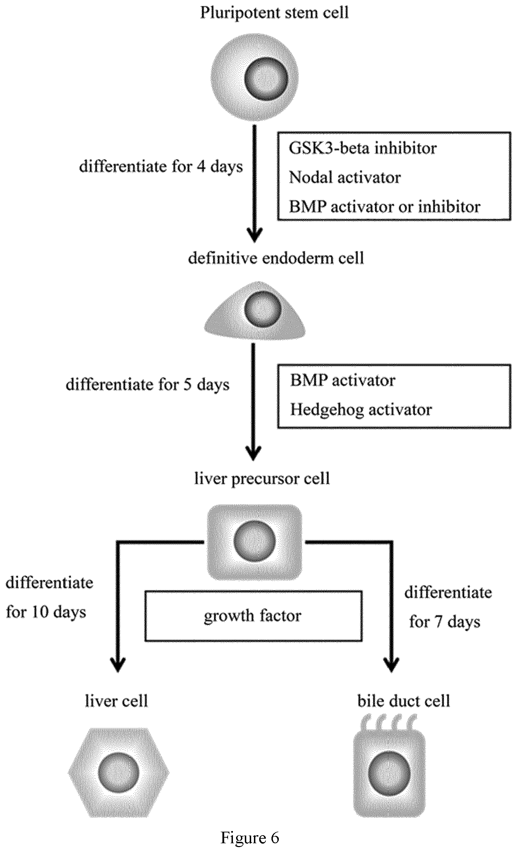

FIG. 6 is a flowchart of the entire experiment according to the present application.

### DETAILED DESCRIPTION

To further elaborate on the technical means adopted and the effects achieved in the present application, the solutions of the present application are further described below through specific examples in conjunction with drawings, but the present application is not limited to the scope of the examples.

### Definition

Embryonic stem cells: stem cells that originate from initial undifferentiated cells of the inner cell mass in the early embryo, can "limitlessly" self-renew *in vitro*, and have the potential to differentiate into cells of three germ layers; in the present application, embryonic stem cells mainly refer to human embryonic stem cell lines HI, numbered as WA01, and H9, numbered as WA09.

Induced pluripotent stem cells: a kind of stem cells that are artificially induced by cell reprogramming technology and have similar characteristics to embryonic stem cells; in the present application, induced pluripotent stem cells mainly refer to induced pluripotent stem cells UC-013, numbered as UiPSC-013, and UC-015, numbered as UiPSC-015, which are obtained by reprogramming urine cells.

Pluripotent stem cells: stem cells capable of differentiating into various types of cells; in the present application, pluripotent stem cells mainly refer to embryonic stem cells and induced pluripotent stem cells.

Basal medium: a nutrient substrate that can support the growth and reproduction of cells and is prepared by combining different nutrients. The basal medium can provide carbohydrates, amino acids, vitamins, trace elements, water and other substances required by cells. Basal mediums that can be used in the present application include, but are not limited to, RPMI 1640, Dulbecco's Modified Eagle Medium, Ham's F12, DMEM/F12, Ham's F-12K Medium, HepatoZYME-SFM, William's E Medium, Waymouth's Medium, and Hepatocyte Culture Medium; wherein the endoderm induction medium is preferably RPMI 1640; and the hepatic progenitor cell induction medium is preferably Hepatozyme-SFM.

Small molecule compounds: mainly refer to chemically synthesized substances, usually with molecular weight of less than 1000; the small molecule compounds used in the present application mainly refer to GSK3-beta inhibitors, BMP inhibitors, Nodal agonists, BMP agonists, and Hedgehog agonists.

GSK3-beta inhibitor: refers to a compound capable of inhibiting GSK3 signaling pathway, including but not limited to CHIR99021 HCL, CHIR99021, and BIO.

BMP inhibitor: refers to a compound capable of inhibiting BMP signaling pathway, including but not limited to dorsomorphin 2HCl, dorsomorphin, and LDN-193189.

Nodal agonist: refers to a compound capable of activating Nodal signaling pathway, including but not limited to IDE1 and IDE2.

BMP agonist: refers to a compound capable of activating BMP signaling pathway, including but not limited to isoliquiritigenin, 4-hydroxychalcone, and kartogenin.

Hedghog agonist: refers to a compound capable of activating Hedghog signaling pathway, including but not limited to Smoothened Agonist HCl.

### General method

### 1) Cryopreservation and resuscitation of human pluripotent stem cells

When the cells grow to 80% to 90% confluence, the culture medium is sucked away, the cells are washed twice with RPMI 1640 culture medium, and a proper amount of Accutase which is restored to room temperature is added to digest the cells at 37 °C; with observation under a microscope, when the cells partially fall off or the intercellular space becomes large, Accutase is sucked away, and RPMI 1640 medium is added to gently pipet the medium to make all the cells fall off; the cells are transferred to a 15 mL centrifuge tube and centrifuged at 200 g for 4 minutes; after the supernatant is discarded, 1 mL of cryopreservation solution CS10 is added to resuspend the cells; a proper amount of cryopreservation solution is added to cause the cell density to be 2×10⁶/mL to 2×10⁷/mL, Y27632 with a final concentration of 5 µM is added, the cells and the added substances are mixed well, and the mixture is sub-packed into cryopreservation tubes, 500 µL per tube, with cell types, passage number, and cryopreservation time marked on the wall of the tube; and the cells are placed in a programmed cooling box which is restored to room temperature, left overnight at ―80 °C, and transferred to a liquid nitrogen tank for long-term storage.

Before stem cells are resuscitated, a culture plate is coated with Matrigel working solution with a volume concentration of 1% and placed at 37 °C for at least 1 hour; the cells are taken out of the liquid nitrogen tank, and after the liquid nitrogen volatilizes, the cells are placed in a 37 °C water bath pot with the cryopreservation tube shaken to quickly dissolve the cells; the cells are transferred from the cryopreservation tube to a 15 mL centrifuge tube, the culture medium is added dropwise, and the centrifuge tube is gently shaken to make the culture medium and the cells fully mixed; 5 mL to 6 mL of culture medium is added and centrifuged at 200 g for 4 minutes; the supernatant is removed and 1 mL of mTeSR is added to resuspend the cells; and a proper amount of culture medium is added until the cell density meets the experimental requirements, Y27632 with a final concentration of 5 µM is added, and the cells are plated in the culture plate and cultured at 37 °C with 5% CO₂.

### 2) Culture and passage of human pluripotent stem cells

Cells are seeded in a culture plate coated by Matrigel with a volume concentration of 1%, and cultured at 37 °C with 5% CO₂, and the mTeSR medium is changed daily.

When the cells grow to 80% to 90% confluence, the culture medium is sucked away, the cells are washed twice with RPMI 1640 culture medium, a proper amount of Accutase which is restored to room temperature is added to digest the cells at 37 °C; with observation under a microscope, when the cells partially fall off or the intercellular space becomes large, Accutase is sucked away, and RPMI 1640 medium is added to gently pipet the medium to make all the cells fall off; and the cells are transferred to a 15 mL centrifuge tube and centrifuged at 200 g for 4 minutes; after the supernatant is discarded, 1 mL of mTeSR medium is added to resuspend the cells; a proper amount of medium is added until the cell density meets the experimental requirements, Y27632 with a final concentration of 5 µM is added, and the cells are plated in the culture plate coated with Matrigel and cultured at 37 °C with 5% CO₂.

### 3) Gene expression detected by RT-PCR

To-be-detected cells are lysed by Trizol, and RNA is extracted and reversely transcribed into cDNA; and the specific genes of hepatic progenitor cells, hepatic cells, and bile duct cells are analyzed by RT-PCR. The primer sequences are shown in Table 2.

**Table 2 Primer sequences used in RT-PCR**

| Gene | Abbr. | 5'→3' | Product length (bp) |
|---|---|---|---|
| Albumin | ALB | SEQ ID NO: 1: F:TGCAACTCTTCGTGAAACCTATG | 135 |
| | | SEQ ID NO: 2: R:ACATCAACCTCTGGTCTCACC | |
| Alpha fetoprotein | AFP | SEQ ID NO: 3: F:GCCACTTGTTGCCAACTCAG | 121 |
| | | SEQ ID NO: 4: R:GGCCAACACCAGGGTTTACT | |
| Aquaporin 1 | AQP1 | SEQ ID NO: 5: F:CTGGGCATCGAGATCATCGG | 158 |
| | | SEQ ID NO: 6: R:ATCCCACAGCCAGTGTAGTCA | |
| Arginase 1 | ARG | SEQ ID NO: 7: F:GTGGAAACTTGCATGGACAAC | 76 |
| | | SEQ ID NO: 8: R:AATCCTGGCACATCGGGAATC | |
| Argininosuccinate synthase 1 | ASS | SEQ ID NO: 9: F:AGGAAAGGGGAACGATCAGGT | 145 |
| | | SEQ ID NO: 10: R:GTGTTGCTTTGCGTACTCCA | |
| CCAAT enhancer binding protein alpha | CEBPα | SEQ ID NO: 11: F:GGTGGACAAGAACAGCAACGA | 140 |
| | | SEQ ID NO: 12: R:GGCGGTCATTGTCACTGGTC | |
| CCAAT enhancer binding protein beta | CEBPβ | SEQ ID NO: 13: F:CGACGAGTACAAGATCCGGC | 186 |
| | | SEQ ID NO: 14: R:TGCTTGAACAAGTTCCGCAG | |
| Cystic fibrosis transmembrane conductance regulator | CFTR | SEQ ID NO: 15: F:AGGACTATGGACACTTCGTGCCTT | 123 |
| | | SEQ ID NO: 16: R:ATTTGGAACCAGCGCAGTGTTGAC | |
| Cytochrome P450 family 2 subfamily C member 9 | CYP2C9 | SEQ ID NO: 17: F:CAGTTGACTTGTTTGGAGCTG | 101 |
| | | SEQ ID NO: 18: R:CTGGACTTTAGCTGTGACCTC | |
| Cytochrome P450 family 2 subfamily D member 6 | CYP2D6 | SEQ ID NO: 19: F:TGGCAAGGTCCTACGCTTC | 189 |
| | | SEQ ID NO: 20: R:GCCACCACTATGCACAGGTT | |
| Cytochrome P450 family 2 subfamily E member 1 | CYP2E1 | SEQ ID NO: 21: F:GTGATGCACGGCTACAAGG | 163 |
| | | SEQ ID NO: 22: R:GGGTGGTCAGGGAAAACCG | |
| Cytochrome P450 family 3 subfamily A member 4 | CYP3A4 | SEQ ID NO: 23: F:GGTGGTGAATGAAACGCTCAG | 103 |
| | | SEQ ID NO: 24: R:CACCCCTTTGGGAATGAACA | |
| Eomesodermin | EOMES | SEQ ID NO: 25: F:CCGCCACCAAACTGAGATGA | 206 |
| | | SEQ ID NO: 26: R:ACATTTTGTTGCCCTGCATGT | |
| Forkhead box A2 | FOXA2 | SEQ ID NO: 27: F:GGAGCAGCTACTATGCAGAGC | 81 |
| | | SEQ ID NO: 28: R:CGTGTTCATGCCGTTCATCC | |
| Glyceraldehyde-3 -phosphate dehydrogenase | GAPDH | SEQ ID NO: 29: F:GGAGCGAGATCCCTCCAAAAT | 197 |
| | | SEQ ID NO: 30: R:GGCTGTTGTCATACTTCTCATGG | |
| Hepatocyte nuclear factor 4 alpha | HNF4α | SEQ ID NO: 31: F:CGAAGGTCAAGCTATGAGGACA | 141 |
| | | SEQ ID NO: 32: R:ATCTGCGATGCTGGCAATCT | |
| Hematopoietically expressed homeobox | HHEX | SEQ ID NO: 33: F:CGCGCTAAATGGAGGAGACT | 103 |
| | | SEQ ID NO: 34: R:TGGGCAAATCTTGCCTCTG | |
| Hepatocyte nuclear factor 6 One cut homeobox 1 | HNF6 | SEQ ID NO: 35: F:GCTTAGCAGCATGCAAAAGG | 147 |
| | | SEQ ID NO: 36: R:GCAATTCTTTGGATGGACGC | |
| Prospero homeobox 1 | PROX1 | SEQ ID NO: 37: F:CAATTTCCACACCGCCAAC | 102 |
| | | SEQ ID NO: 38: R:TCAGTGGAACTGGCCATCTG | |
| Serpin family A member 1 | AAT | SEQ ID NO: 39: F:GATCAACGATTACGTGGAGAAGG | 207 |
| | | SEQ ID NO: 40: R:CCTAAACGCTTCATCATAGGCA | |
| SRY-box 4 | SOX4 | SEQ ID NO: 41: F:AGCGACAAGATCCCTTTCATTC | 109 |
| | | SEQ ID NO: 42: R:CGTTGCCGGACTTCACCTT | |
| SRY-box 9 | SOX9 | SEQ ID NO: 43: F:AGCGAACGCACATCAAGAC | 85 |
| | | SEQ ID NO: 44: R:CTGTAGGCGATCTGTTGGGG | |

### 4) Detection of protein expression by immunofluorescence

Cell fixation: To-be-detected cells are fixed with 4% paraformaldehyde (PFA) and left overnight at 4 °C, PFA is discarded, and PBS is add to wash the cells three times, each time for 5 minutes.

Blocking and permeablization: PBS is discarded, PBS containing 10% FBS and 0.2% Triton is added, the mixture is treated at room temperature for 1 hour, the blocking/permeablization solution is discarded, and PBS is added to wash the cells three times, each time for 5 minutes.

Incubation of primary antibodies: According to the antibody instruction, an appropriate dilution multiple is selected, the primary antibodies are diluted and incubated overnight at 4 °C; and the primary antibodies are discarded, and the cells are washed three times with PBS, each time for 15 minutes.

Incubation of secondary antibodies: According to the species of the primary antibodies, appropriate secondary antibodies are selected, diluted, and incubated at room temperature for 1 hour; and the secondary antibodies are discarded, and the cells are washed with PBS three times, each time for 15 minutes.

Nuclear staining: The cells are incubated at room temperature for 5 minutes with DAPI with an appropriate concentration, DAPI is discarded, and the cells are washed three times with PBS, each time for 15 minutes.

Observation: The cells are observed under a fluorescence microscope.

### 5) Detection of protein expression by flow cytometry

Cell digestion: The culture medium of cells to-be-detected is discarded, the cells are washed three times with PBS, an appropriate amount of Accutase or pancreatin which is restored to room temperature is added to digest the cells at 37 °C. With observation under a microscope, when the cells are digested into single cells, 10% FBS is added to terminate digestion, the cells are collected into an Ep tube, centrifuged at 250 g for 4 minutes, and the supernatant discarded.

Cell fixation and permeablization: When the intracellular protein is to be detected, the cells need to be fixed and be subjected to permeablization, 50 µL of PBS is added to resuspend the cells, 250 µL of Fixation/Permeablization solution is added, the mixture is treated on ice for 20 minutes, PBS is added until the mixture becomes 1 mL, the mixture is centrifuged at 250g for 4 minutes, and the supernatant is discarded.

Blocking: 10% FBS is added to resuspend the cells until the mixture becomes 1 mL, the cells are incubated at room temperature for 10 minutes and centrifuged at 250 g for 4 minutes, and the supernatant is discarded.

Washing: PBS is added to resuspend the cells until the mixture becomes 1 mL, the cells are centrifuged at 250g for 4 minutes, and the supernatant is discarded; this step is repeated once.

Incubation of antibodies: according to the instruction, the antibodies are diluted and incubated on ice for 30 minutes, PBS is added to resuspend the cells until the mixture becomes 1 mL, the cells are centrifuged at 250 g for 4 minutes, and the supernatant is discarded.

Resuspending: The cells are resuspended with PBS, filtered before loaded onto the flow cytometer, and detected by the flow cytometer.

Data analysis: Data is analyzed by using FLowjo.

### Example 1 Obtaining of definitive endoderm cells

Day 0: When the cells grew to 50% to 80% confluence, the directional differentiation experiment started to be carried out. The mTeSR medium was discarded. The cells were washed twice with the basal medium RPMI 1640 and added with an endoderm induction medium I formed by adding insulin-free B27 with the final concentration of 2%, 3 µM of CHIR99021, 1 µM of IDE1, and 2.5 µM of isoliquiritigenin into RPMI 1640 which served as the basal medium.

Day 1 and 2: The medium was discarded, and an endoderm induction medium II was added, in which the endoderm induction medium II was formed by adding insulin-free B27 with the final concentration of 2%, 1 µM of CHIR99021, 1 µM of IDE1, and 2.5 µM of isoliquiritigenin into RPMI 1640 which served as the basal medium.

Day 3: The medium was discarded, and an endoderm induction medium III was added, in which the endoderm induction medium III was formed by adding insulin-free B27 with the final concentration of 2%, 1 µM of CHIR99021, 1 µM of IDE1, and 0.2 µM of dorsomorphin 2HCl into RPMI 1640 which served as the basal medium.

Day 4: Definitive endoderm cells were obtained for identification or subsequent differentiation.

### Example 2 Obtaining of hepatic progenitor cells

Day 4 to 8: The following steps were repeated every day during this period of time: the medium was discarded, and a hepatic progenitor medium was added, in which the hepatic progenitor medium was formed by adding B27 with the final concentration of 2%, 2.5 µM of isoliquiritigenin, 2.5 µM of 4-hydroxychalcone, and 0 to 2 µM of Smoothened Agonist HCl into HepatoZYME-SFM which served as the basal medium.

Day 9: Hepatic progenitor cells were obtained for identification or subsequent differentiation.

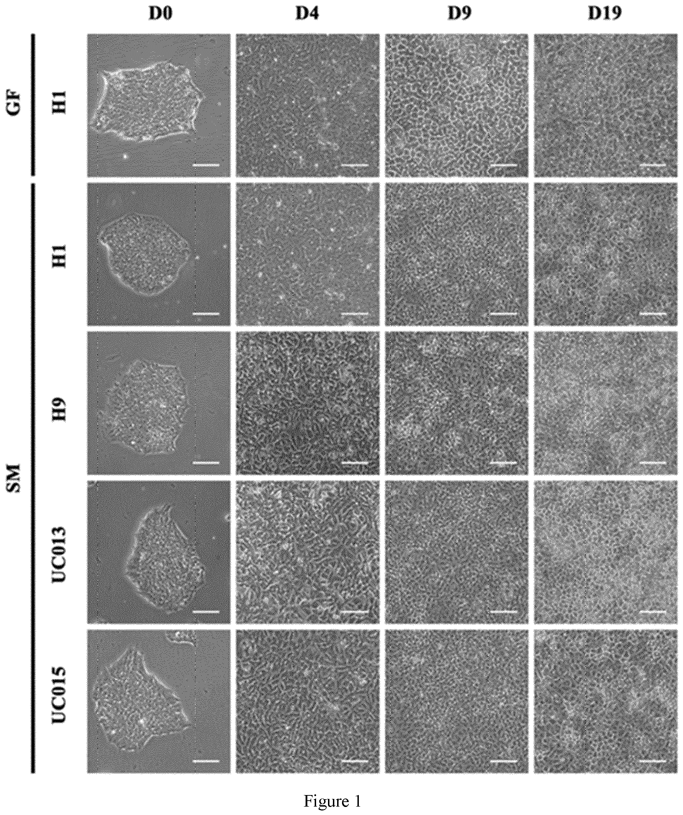

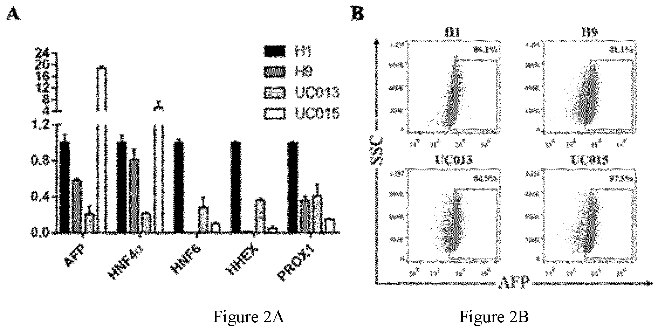

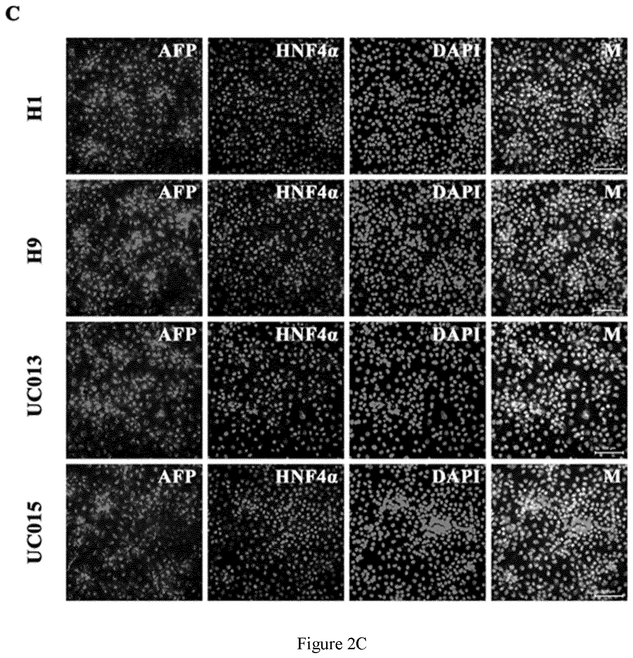

Identification of marker genes and proteins: Genes specifically expressed by hepatic progenitor cells were detected by RT-PCR, such as *AFP* and *HNF4α*; protein AFP specifically expressed by hepatic progenitor cells was detected by flow cytometry; and proteins AFP and HNF4α specifically expressed by hepatic progenitor cells were detected by immunofluorescence. The morphological changes of cells during the differentiation process are shown in FIG. 1, and the results of hepatic progenitor cells (HPCs) induced by small molecule compounds are shown in FIGs. 2A and 2B.

According to the preceding identification results, hepatic progenitor cells with a positive rate of more than 80% can be obtained by using the small molecule induction program.

### Example 3 Validation of differentiation potential of hepatic progenitor cells

### 1) Differentiation of hepatic progenitor cells into hepatic cells

Day 9 to 13: The following steps were repeated every day during this period of time: the medium was discarded, and a liver induction medium I was added, in which the liver induction medium I was formed by adding B27 with the final concentration of 2%, 20 ng/mL HGF, and 20 ng/mL KGF into HepatoZYME-SFM which served as the basal medium.

Day 14 to 18: The following steps were repeated every day during this period of time: the medium was discarded, and a liver induction medium II was added, in which the liver induction medium II was formed by adding B27 with the final concentration of 2%, 0.1 µM of dexamethasone, and 10 ng/mL OSM into HepatoZYME-SFM which served as the basal medium.

Day 19: Hepatic cells were obtained for identification.

The morphological changes of cells during the differentiation process are shown in FIG. 1.

Identification of marker genes and proteins: genes specifically expressed by hepatic cells were detected by RT-PCR, such as *ALB* and *AAT*; and proteins ALB and AAT specifically expressed by hepatic cells were detected by immunofluorescence.

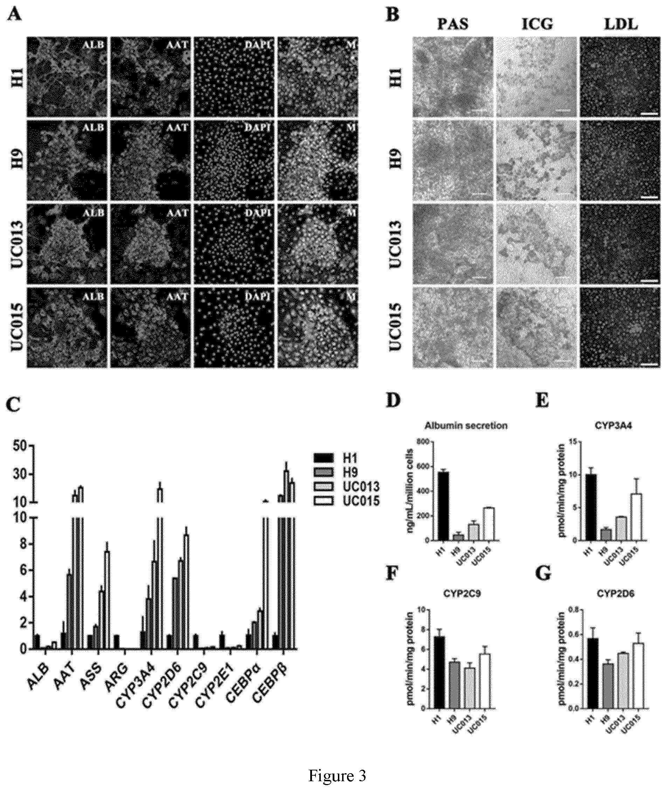

Function identification: The glycogen synthesis ability was detected by PAS staining; the liver function was detected by ICG and LDL uptake test; the albumin expression ability was detected by ELISA test; and CYP3A4, CYP2C9, and CYP2D6 were detected by mass spectrometry. The results are shown in FIGs. 3A to 3G.

According to the preceding identification results, hepatic progenitor cells induced by small molecules can differentiate into functional hepatic cells in suitable induction conditions.

### 2) Differentiation of hepatic progenitor cells into bile duct cells

Day 9 to 15: The following steps were repeated every day during this period of time: the medium was discarded, and a bile duct induction medium was added, in which the bile duct induction medium was formed by adding B27 with the final concentration of 2%, 50 ng/mL EGF, 20 ng/mL HGF, 5 ng/mL TGFβ1 into the William's E medium which served as the basal medium.

Day 16: Bile duct cells were obtained for identification.

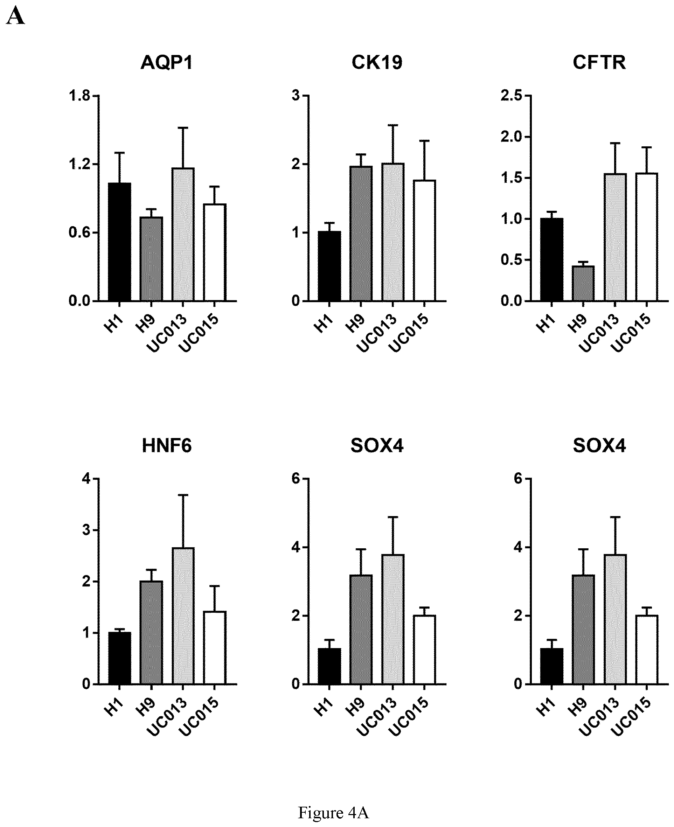

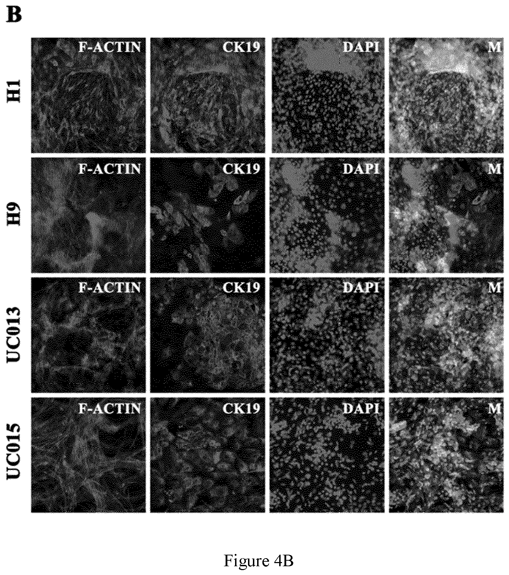

Identification of marker genes and proteins: genes specifically expressed by bile duct cells were detected by RT-PCR, such as CK19 and CFTR; and proteins F-ACTIN and CK19 specifically expressed by bile duct cells were detected by immunofluorescence. The results are shown in FIGs. 4A and 4B.

According to the preceding identification results, hepatic progenitor cells induced by small molecules can differentiate into bile duct cells in suitable induction conditions.

### Example 4 Transplantation of hepatic progenitor cells to repair mouse liver

Construction of mouse models of acute liver injury: Dimethylnitrosamine (DMN) was given at 8 mg/kg of body weight by intraperitoneal injection for two consecutive days to make immunodeficient mice NSI (NOD/SCID/IL2rg-/-) suffer from acute liver injury. Three days after the models were constructed, the hepatic progenitor cells were transplanted.

Transplantation of hepatic progenitor cells: 1 × 10⁶ of EPCAM+ & C-KIT- hepatic progenitor cells were injected into the spleen of mice. After one week of feeding, the livers were taken for analysis and examination.

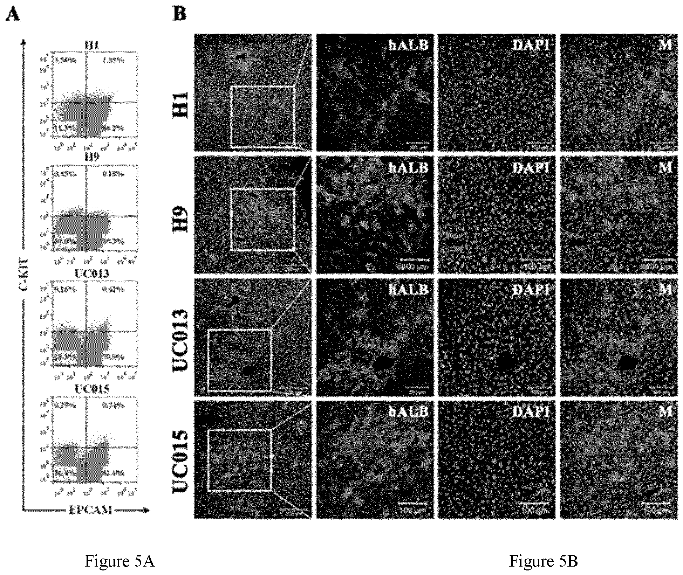

The mouse livers were detected by immunofluorescence. The results are shown in FIGs. 5A and 5B. The results show that the mouse livers had hepatic cells expressing human albumin, which indicates that small molecule-induced hepatic progenitor cells can further differentiate into hepatic cells in the mouse liver.

In summary, the present application provides a composition and use thereof. The composition is used for preparing a medium for inducing differentiation of human pluripotent stem cells into hepatic progenitor cells. By means of screening active components, optimizing the composition ratio, and adding a GSK3-beta inhibitor, a Nodal agonist, a BMP agonist, a BMP inhibitor, and a Hedgehog agonist into differentiating cells, human pluripotent stem cells are induced to differentiate into hepatic progenitor cells. The process is simple and efficient, the content of positive cells is high, and the cost of cell differentiation is reduced. The composition and application thereof can be used for research and application in drug development and regenerative medicinal treatment and have broad application prospects.

The applicant has stated that although the detailed method of the present application is described through the examples described above, the present application is not limited to the detailed method described above, which means that implementation of the present application does not necessarily depend on the detailed method described above. It should be apparent to those skilled in the art that any improvements made to the present application, equivalent replacements of raw materials of the product of the present application, additions of adjuvant ingredients to the product of the present application, and selections of specific manners, etc., all fall within the protection scope and the disclosed scope of the present application.

## Claims

1. A composition, comprising a basal medium, and further comprising any one or a combination of at least two of a GSK3-beta inhibitor, a Nodal agonist, a BMP agonist, a BMP inhibitor, a Hedgehog agonist or a serum substitute.

2. The composition according to claim 1, wherein the basal medium comprises any one or a combination of at least two of RPMI 1640, Dulbecco's Modified Eagle Medium, Ham's F12, DMEM/F12, Ham's F-12K Medium, HepatoZYME-SFM, William's E Medium, Waymouth's Medium or Hepatocyte Culture Medium.

3. The composition according to claim 1 or 2, wherein the GSK3-beta inhibitor comprises any one or a combination of at least two of CHIR99021 HCL, CHIR99021 or BIO, preferably CHIR99021 HCL;
preferably, the concentration of CHIR99021 HCL is 0.5 µM to 4.5 µM;
preferably, the Nodal agonist comprises IDE1 and/or IDE2;
preferably, the concentration of IDE1 is 0.5 µM to 4 µM;
preferably, the BMP agonist comprises any one or a combination of at least two of isoliquiritigenin, 4-hydroxychalcone or kartogenin;
preferably, the concentration of the BMP agonist is from 1 µM to 20 µM.

4. The composition according to any one of claims 1 to 3, wherein the BMP inhibitor comprises any one or a combination of at least two of dorsomorphin 2HCl, dorsomorphin or LDN-193189;
preferably, the concentration of the BMP inhibitor, dorsomorphin 2HCl, is 0 to 1 µM;
preferably, the Hedgehog agonist comprises Smoothened Agonist HCl;
preferably, the concentration of the Hedgehog agonist is 0.2 µM to 1 µM;
preferably, the serum substitute comprises insulin-free B27 and/or insulin-containing B27;
preferably, the volume concentration of the serum substitute is 0% to 3%.

5. Use of the composition according to any one of claims 1 to 4 in the preparation of a cell culture medium for inducing differentiation of human pluripotent stem cells into hepatic progenitor cells.

6. The use according to claim 5, wherein the human pluripotent stem cells comprise a human embryonic stem cell line and a human induced pluripotent stem cell line;
preferably, the human embryonic stem cell line comprises WA01 and/or WA09;
preferably, the human induced pluripotent stem cell line comprises UiPSC-013 and/or UiPSC-015.

7. A cell culture medium for inducing differentiation of human pluripotent stem cells into hepatic progenitor cells, wherein the cell culture medium is prepared from the composition according to any one of claims 1 to 4.

8. The cell culture medium according to claim 7, wherein the cell culture medium comprises any one or a combination of at least two of an endoderm induction medium I, an endoderm induction medium II, an endoderm induction medium III or a hepatic progenitor cell induction medium;
preferably, the endoderm induction medium I contains insulin-free B27 with a volume concentration of 0 to 3%, CHIR99021 HCL with a concentration of 1 µM to 4.5 µM, IDE1 with a concentration of 0.5 µM to 4 µM, isoliquiritigenin with a concentration of 1 µM to 20 µM, and a basal medium;
preferably, the endoderm induction medium II contains insulin-free B27 with a volume concentration of 0 to 3%, CHIR99021 HCL with a concentration of 0.5 µM to 3 µM, IDE1 with a concentration of 0.5 µM to 4 µM, isoliquiritigenin with a concentration of 1 µM to 20 µM, and a basal medium;
preferably, the endoderm induction medium III contains insulin-free B27 with a volume concentration of 0 to 3%, CHIR99021 HCL with a concentration of 3 µM to 4.5 µM, IDE1 with a concentration of 0.5 µM to 4 µM, dorsomorphin 2HCl with a concentration of 0 to 1 µM, and a basal medium;
preferably, the hepatic progenitor medium contains B27 with a volume concentration of 0 to 3%, isoliquiritigenin with a concentration of 1 µM to 20 µM, 4-hydroxychalcone with a concentration of 1 µM to 20 µM, Smoothened Agonist HCl with a concentration of 0 to 2 µM, and a basal medium.

9. A method for inducing differentiation of pluripotent stem cells into hepatic progenitor cells, wherein the method is performed by adopting the cell culture medium according to claim 7 or 8.

10. The method according to claim 9, comprising the following steps:
(1) culturing pluripotent stem cells by using an endoderm induction medium I;
(2) culturing the cells obtained in step (1) by using an endoderm induction medium II;
(3) culturing the cells obtained in step (2) by using an endoderm induction medium III;
(4) culturing the cells obtained in step (3) by using a hepatic progenitor cell induction medium to obtain hepatic progenitor cells; and
(5) detecting and analyzing the obtained hepatic progenitor cells;
preferably, a method for the detection and analysis in step (5) is to check the expression of a hepatic progenitor cell marker by fluorescence quantitative PCR, immunofluorescence or flow cytometry;
preferably, the hepatic progenitor cell marker comprises any one or a combination of at least two of AFP, HNF4α or EPCAM


SEQUENCE LISTING

<110>  GUANGZHOU INSTITUTES OF BIOMEDICINE AND HEALTH CHINESE
       ACADEMY OF SCIENCES

<120>  COMPOSITION AND APPLICATION THEREOF

<130>  19DX1536FPPC

<150>  CN201910091150.0
<151>  2019-01-30

<160>  44

<170>  PatentIn version 3.3

<210>  1
<211>  23
<212>  DNA
<213>  Artificial sequence

<400>  1
tgcaactctt cgtgaaacct atg                                              23


<210>  2
<211>  21
<212>  DNA
<213>  Artificial sequence

<400>  2
acatcaacct ctggtctcac c                                                21


<210>  3
<211>  20
<212>  DNA
<213>  Artificial sequence

<400>  3
gccacttgtt gccaactcag                                                  20


<210>  4
<211>  20
<212>  DNA
<213>  Artificial sequence

<400>  4
ggccaacacc agggtttact                                                  20


<210>  5
<211>  20
<212>  DNA
<213>  Artificial sequence

<400>  5
ctgggcatcg agatcatcgg                                                    20


<210>  6
<211>  21
<212>  DNA
<213>  Artificial sequence

<400>  6
atcccacagc cagtgtagtc a                                                  21


<210>  7
<211>  21
<212>  DNA
<213>  Artificial sequence

<400>  7
gtggaaactt gcatggacaa c                                                  21


<210>  8
<211>  21
<212>  DNA
<213>  Artificial sequence

<400>  8
aatcctggca catcgggaat c                                                  21


<210>  9
<211>  21
<212>  DNA
<213>  Artificial sequence

<400>  9
aggaaagggg aacgatcagg t                                                  21


<210>  10
<211>  20
<212>  DNA
<213>  Artificial sequence

<400>  10
gtgttgcttt gcgtactcca                                                    20


<210>  11
<211>  21
<212>  DNA
<213>  Artificial sequence

<400>  11

ggtggacaag aacagcaacg a                                                21

<210>  12
<211>  20
<212>  DNA
<213>  Artificial sequence

<400>  12
ggcggtcatt gtcactggtc                                                   20

<210>  13
<211>  20
<212>  DNA
<213>  Artificial sequence

<400>  13
cgacgagtac aagatccggc                                                   20

<210>  14
<211>  20
<212>  DNA
<213>  Artificial sequence

<400>  14
tgcttgaaca agttccgcag                                                   20

<210>  15
<211>  24
<212>  DNA
<213>  Artificial sequence

<400>  15
aggactatgg acacttcgtg cctt                                              24

<210>  16
<211>  24
<212>  DNA
<213>  Artificial sequence

<400>  16
atttggaacc agcgcagtgt tgac                                              24

<210>  17
<211>  21
<212>  DNA
<213>  Artificial sequence

<400>  17
cagttgactt gtttggagct g                                                 21

<210>  18
<211>  21
<212>  DNA
<213>  Artificial sequence

<400>  18
ctggacttta gctgtgacct c                                                21


<210>  19
<211>  19
<212>  DNA
<213>  Artificial sequence

<400>  19
tggcaaggtc ctacgcttc                                                   19


<210>  20
<211>  20
<212>  DNA
<213>  Artificial sequence

<400>  20
gccaccacta tgcacaggtt                                                  20


<210>  21
<211>  19
<212>  DNA
<213>  Artificial sequence

<400>  21
gtgatgcacg gctacaagg                                                   19


<210>  22
<211>  19
<212>  DNA
<213>  Artificial sequence

<400>  22
gggtggtcag ggaaaaccg                                                   19


<210>  23
<211>  21
<212>  DNA
<213>  Artificial sequence

<400>  23
ggtggtgaat gaaacgctca g                                                21


<210>  24

<211>  20
<212>  DNA
<213>  Artificial sequence

<400>  24
cacccctttg ggaatgaaca                                                   20


<210>  25
<211>  20
<212>  DNA
<213>  Artificial sequence

<400>  25
ccgccaccaa actgagatga                                                   20


<210>  26
<211>  21
<212>  DNA
<213>  Artificial sequence

<400>  26
acattttgtt gccctgcatg t                                                 21


<210>  27
<211>  21
<212>  DNA
<213>  Artificial sequence

<400>  27
ggagcagcta ctatgcagag c                                                 21


<210>  28
<211>  20
<212>  DNA
<213>  Artificial sequence

<400>  28
cgtgttcatg ccgttcatcc                                                   20


<210>  29
<211>  21
<212>  DNA
<213>  Artificial sequence

<400>  29
ggagcgagat ccctccaaaa t                                                 21


<210>  30
<211>  23
<212>  DNA

<213>  Artificial sequence

<400>  30
ggctgttgtc atacttctca tgg                                              23


<210>  31
<211>  22
<212>  DNA
<213>  Artificial sequence

<400>  31
cgaaggtcaa gctatgagga ca                                               22


<210>  32
<211>  20
<212>  DNA
<213>  Artificial sequence

<400>  32
atctgcgatg ctggcaatct                                                  20


<210>  33
<211>  20
<212>  DNA
<213>  Artificial sequence

<400>  33
cgcgctaaat ggaggagact                                                  20


<210>  34
<211>  19
<212>  DNA
<213>  Artificial sequence

<400>  34
tgggcaaatc ttgcctctg                                                   19


<210>  35
<211>  20
<212>  DNA
<213>  Artificial sequence

<400>  35
gcttagcagc atgcaaaagg                                                  20


<210>  36
<211>  20
<212>  DNA
<213>  Artificial sequence

<400>  36
gcaattcttt ggatggacgc                                                    20

<210>  37
<211>  19
<212>  DNA
<213>  Artificial sequence

<400>  37
caatttccac accgccaac                                                     19

<210>  38
<211>  20
<212>  DNA
<213>  Artificial sequence

<400>  38
tcagtggaac tggccatctg                                                    20

<210>  39
<211>  23
<212>  DNA
<213>  Artificial sequence

<400>  39
gatcaacgat tacgtggaga agg                                                23

<210>  40
<211>  22
<212>  DNA
<213>  Artificial sequence

<400>  40
cctaaacgct tcatcatagg ca                                                 22

<210>  41
<211>  22
<212>  DNA
<213>  Artificial sequence

<400>  41
agcgacaaga tccctttcat tc                                                 22

<210>  42
<211>  19
<212>  DNA
<213>  Artificial sequence

<400>  42
cgttgccgga cttcacctt                                                     19

<210>  43
<211>  19
<212>  DNA
<213>  Artificial sequence

<400>  43
agcgaacgca catcaagac                                                    19

<210>  44
<211>  20
<212>  DNA
<213>  Artificial sequence

<400>  44
ctgtaggcga tctgttgggg                                                   20